# EUROPEAN PATENT SPECIFICATION

(11) **EP 0 429 314 B1**
(45) Date of publication and mention of the grant of the patent: **01.02.1995**
(21) Application number: 90312792.6
(22) Date of filing: 23.11.1990
(51) Int. Cl.: C07C 217/20, A01N 33/10

(54) **Phenoxyalkylamine and agricultural and horticultural bactericide**
Phenoxyalkylamin und Bakterizid für Landwirtschaft und Gartenbau
Phénoxyalcoylamine et bactéricide pour l'agriculture et l'horticulture

(30) Priority: 24.11.1989 JP 303238/89
(43) Date of publication of application: 29.05.1991
(73) Proprietor: MITSUBISHI GAS CHEMICAL COMPANY, INC., Tokyo, 100 (JP)
(72) Inventor: Mitsunori, Oda, c/o Niigata Research Laboratory, 182, Shinwari, Tayuhama, Niigata-shi (JP); Kazutoshi, Kikkawa, c/o Niigata Research, 182, Shinwari, Tayuhama, Niigata-shi (JP); Akinori, Tanaka, c/o Niigata Research Laboratory, 182, Shinwari, Tayuhama, Niigata-shi (JP); Satoko, Imaruoka, c/o Niigata Research Laboratory, 182, Shinwari, Tayuhama, Niigata-shi (JP); Shigeo, Yoshinaka, c/o Niigata Research Laboratory, 182, Shinwari, Tayuhama, Niigata-shi (JP)
(74) Representative: Cresswell, Thomas Anthony

(56) References cited:
- EP-A- 0 269 363
- DE-B- 1 020 031
- US-A- 3 567 723
- US-A- 3 677 735
- US-A- 3 729 511

## Description

The present invention relates to an agricultural and horticultural bactericide.

Inorganic copper-containing agents, organic copper-containing agents and antibiotic agents such as streptomycin have been used as bactericides for pathogenic bacteria causing blights in agricultural plants. However, these conventional bactericides are ineffective and phytotoxic. Accordingly, development of a bactericide having strong bacteriostatic and bactericidal actions (collectively called "antibacterial action" hereinafter) and having a reduced phytotoxicity is needed.

US-A-3 729 511 discloses halophenoxymethylamines which are useful in the control of bacteria. US-A-3 677 735 discloses a method for the control of aquatic plant life which comprises contacting the plant life with a phytotoxic amount of a substituted phenoxyamine.

Unexpectedly it has been found that phenoxyalkylamines and salts thereof have bacteriostatic and bacterial actions to pathogenic bacteria causing blights in agricultural and horticultural plants.

The present invention therefore provides the use of agricultural or horticultural bactericide comprising an active ingredient which is a phenoxyalkylamine of formula (I) 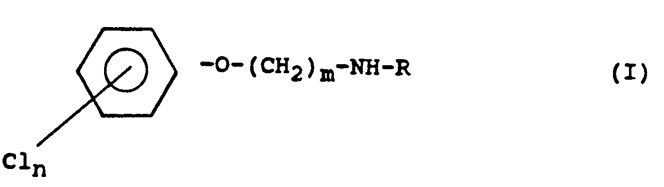 wherein R is a linear saturated hydrocarbon having 7 to 12 carbon atoms, n is an integer of from 1 to 5 and m is an integer of from 2 to 4, or a mineral acid salt thereof, for preventing or curing a plant disease.

The phenoxyalkylamine of the present invention represented by general formula I is prepared, for example, by the following reaction:

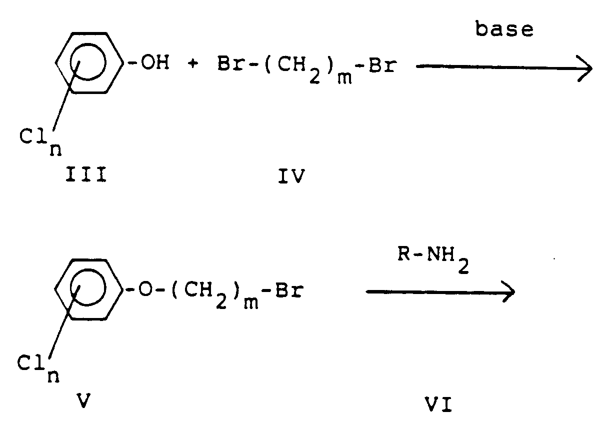

Namely, phenol III is reacted with dibromoalkane IV in the presence of a base such as sodium hydroxide in a solvent such as water, an alcohol, dimethylsulfoxide or dimethylformamide to convert the phenol III to compound V, and this compound V is reacted with amine VI in the presence of a hydrogen bromide scavenger in a solvent such as an alcohol, dimethylsulfoxide or dimethylformamide to obtain the phenoxyalkylamine I of the present invention.

The phenoxyalkylamine salt of the present invention is prepared, for example, by reacting the above- mentioned phenoxyalkylamine I with a mineral acid.

The mineral acid used is not particularly critical, and the mineral acid can be used in either the gaseous state or the liquid state. As typical instances, there can be mentioned hydrochloric acid, hydrobromic acid, hydroiodic acid, sulfuric acid, nitric acid and phosphoric acid.

In the preparation of the salt, use of a reaction solvent is not absolutely indispensable, but a solvent ordinarily used as a reaction solvent, for example, an alcohol, an ether or an ester, or water, can be used as the reaction solvent.

The reaction solvent is not particularly critical, but when a gaseous mineral acid is used, if the reaction is carried out at a low temperature such as 0 _{°} C, the loss of the mineral acid can be reduced.

The phenoxyalkylamine salt of the present invention is solid at room temperature or normal temperature, and the salt can be recovered from the reaction product liquid by ordinary solid-liquid separating means such as filtration or centrifugal separation and if desired, the recovered crystal can be purified by washing or recrystallization from an alcohol or water.

The physical properties of the phenoxyalkylamine represented by general formula I and the phenoxyalkylamine salt are shown in Tables 1 and 2, respectively.

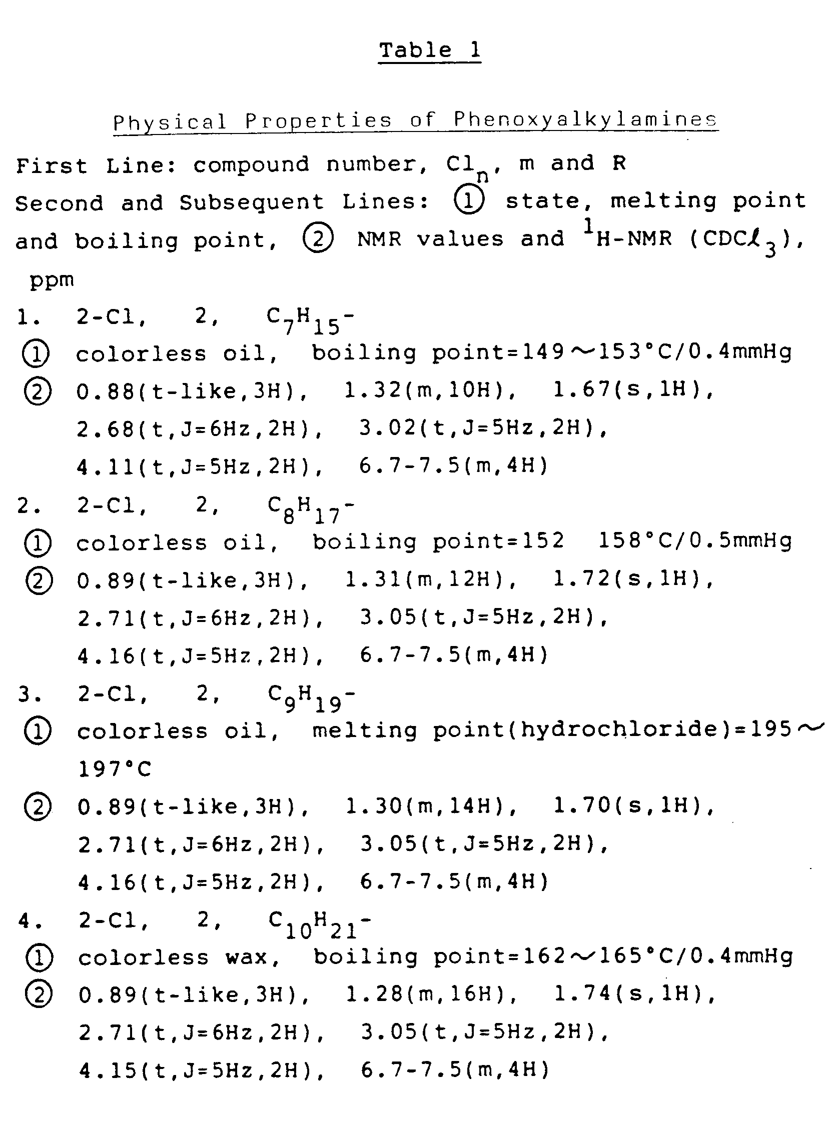

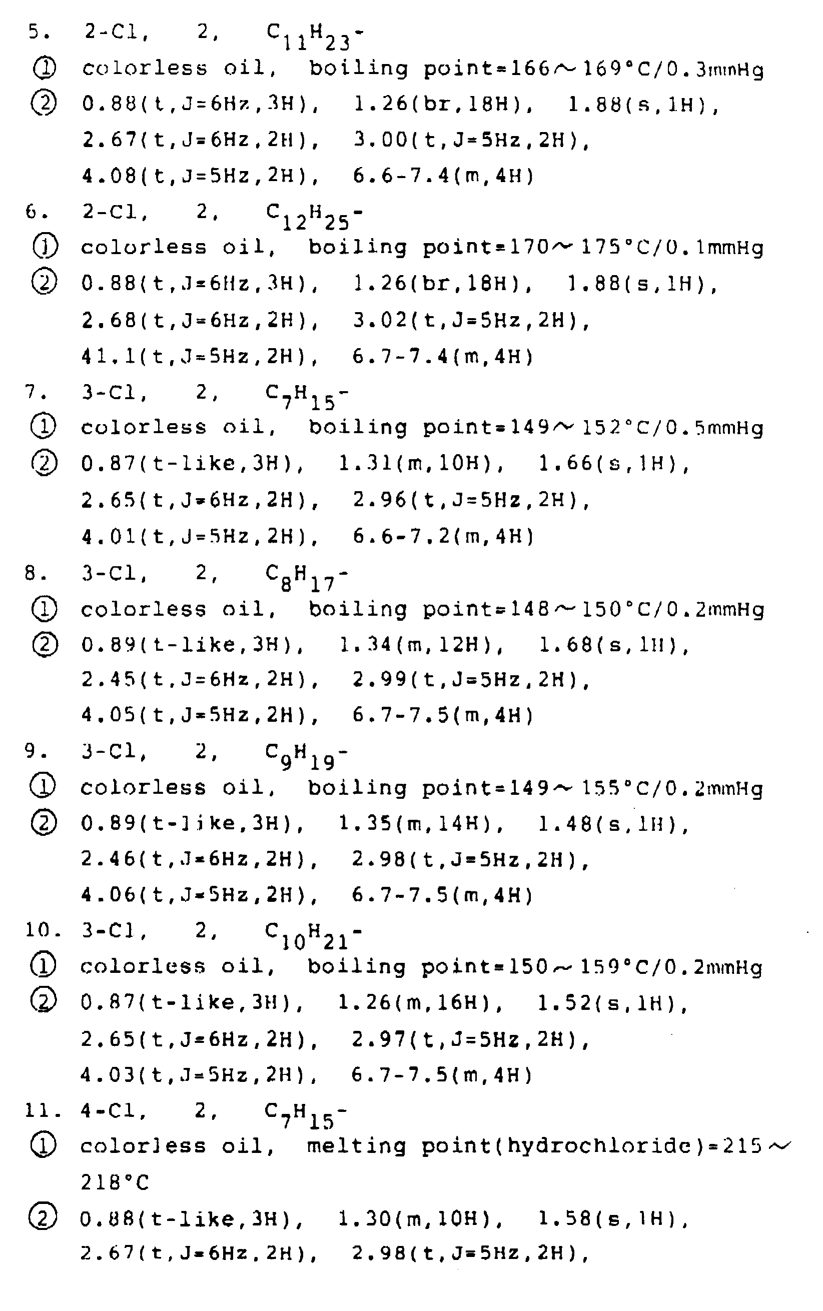

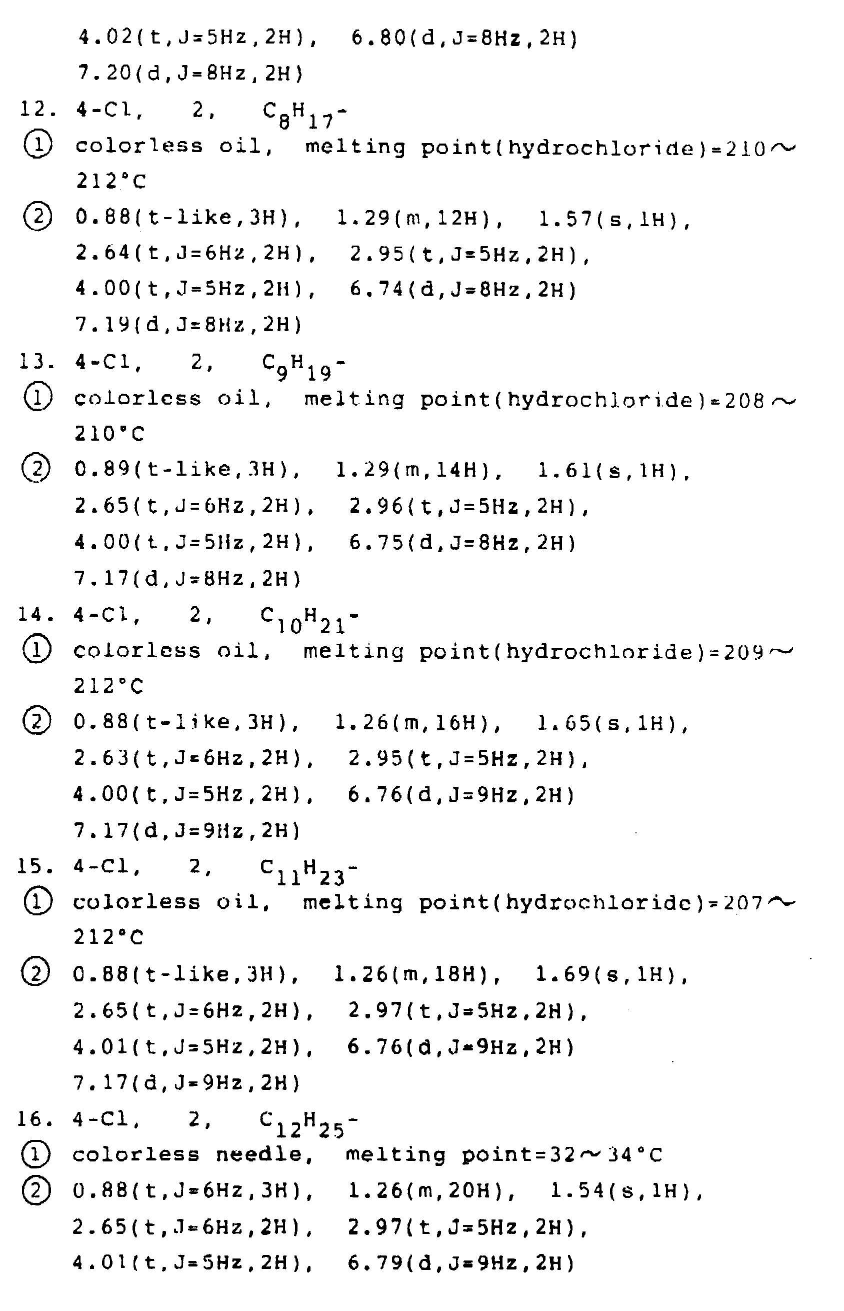

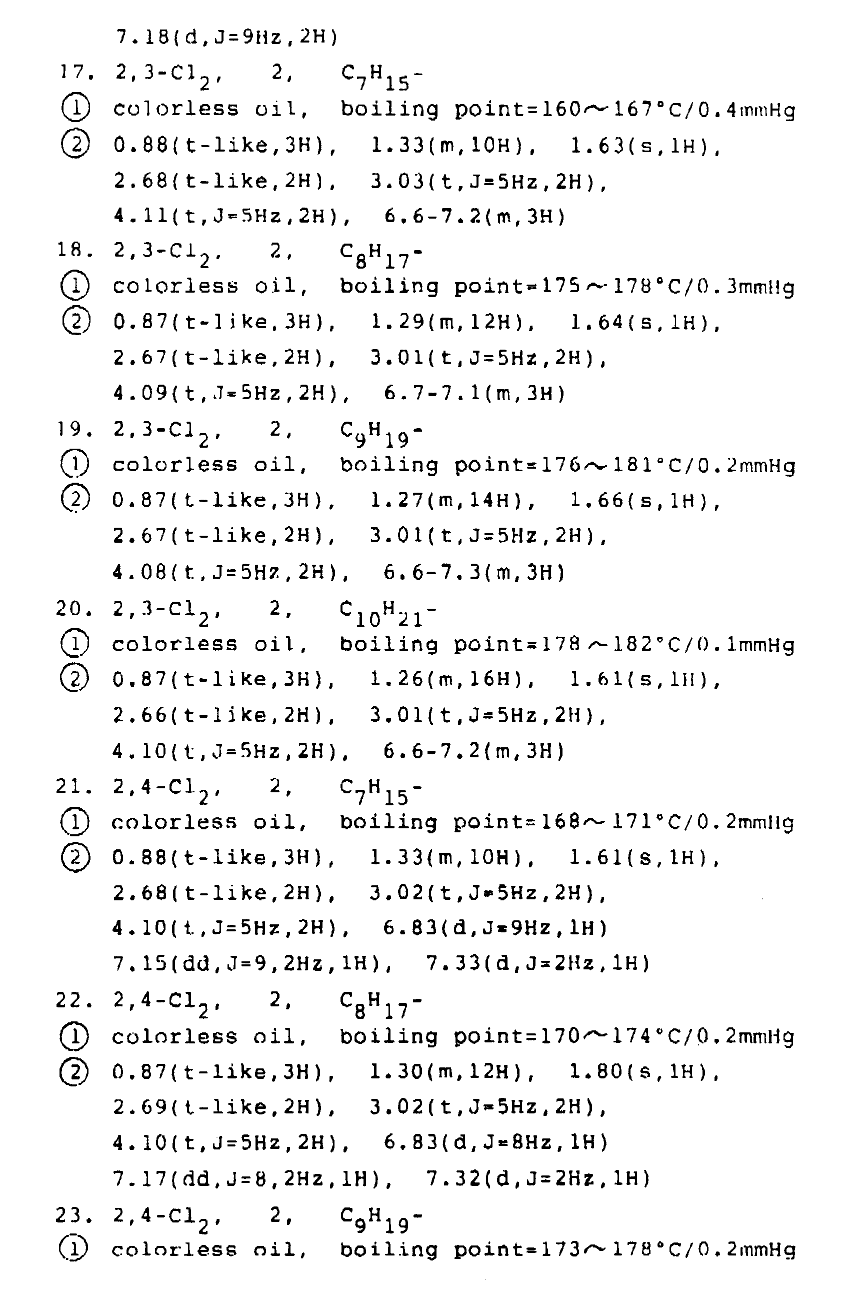

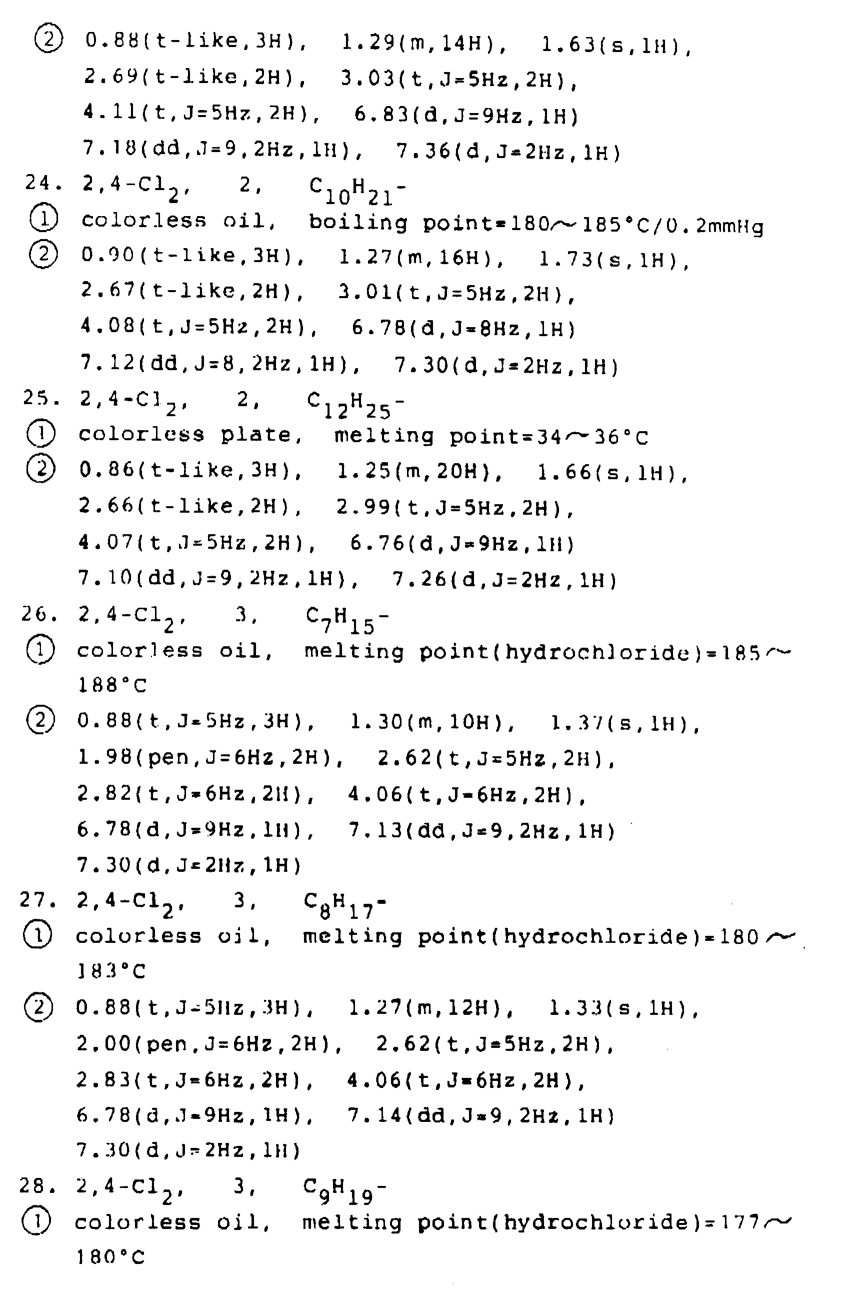

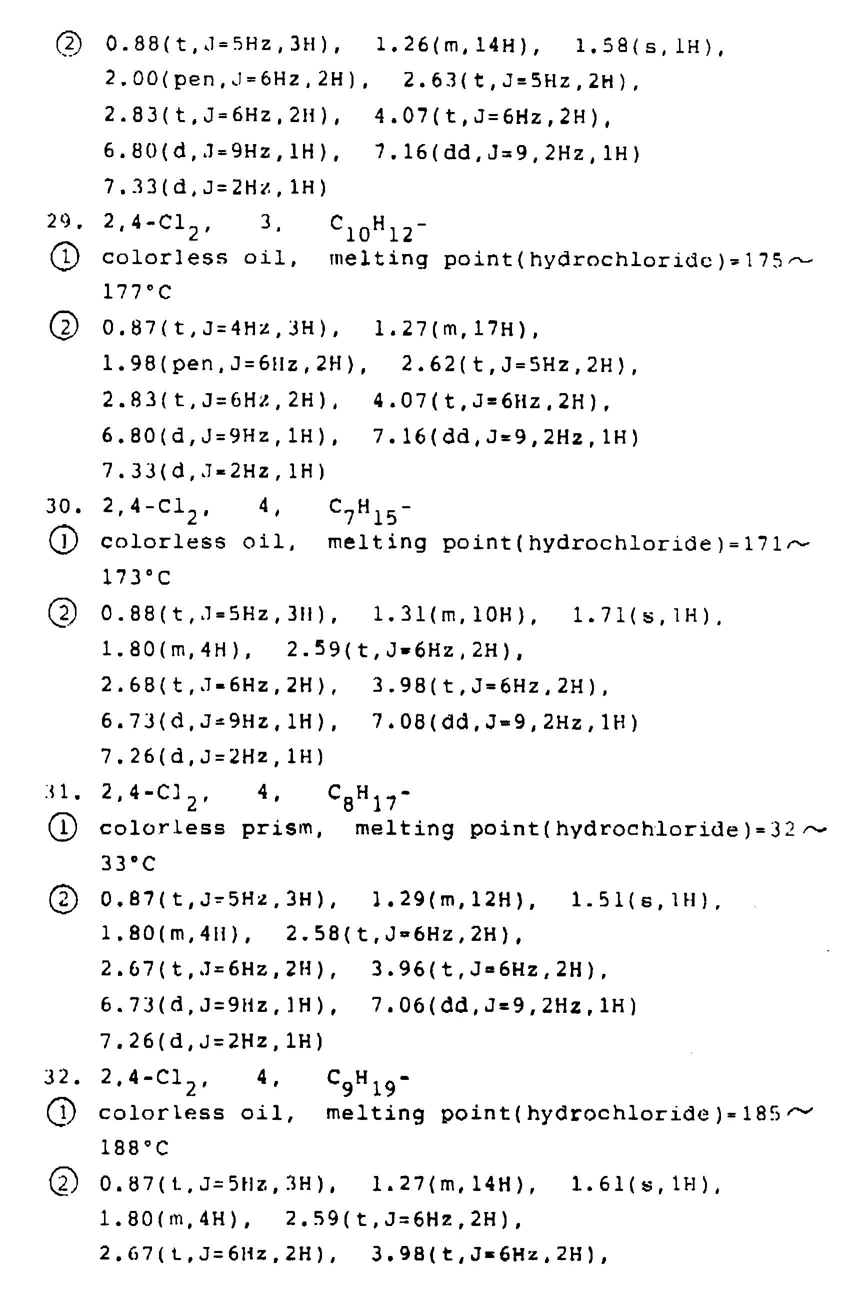

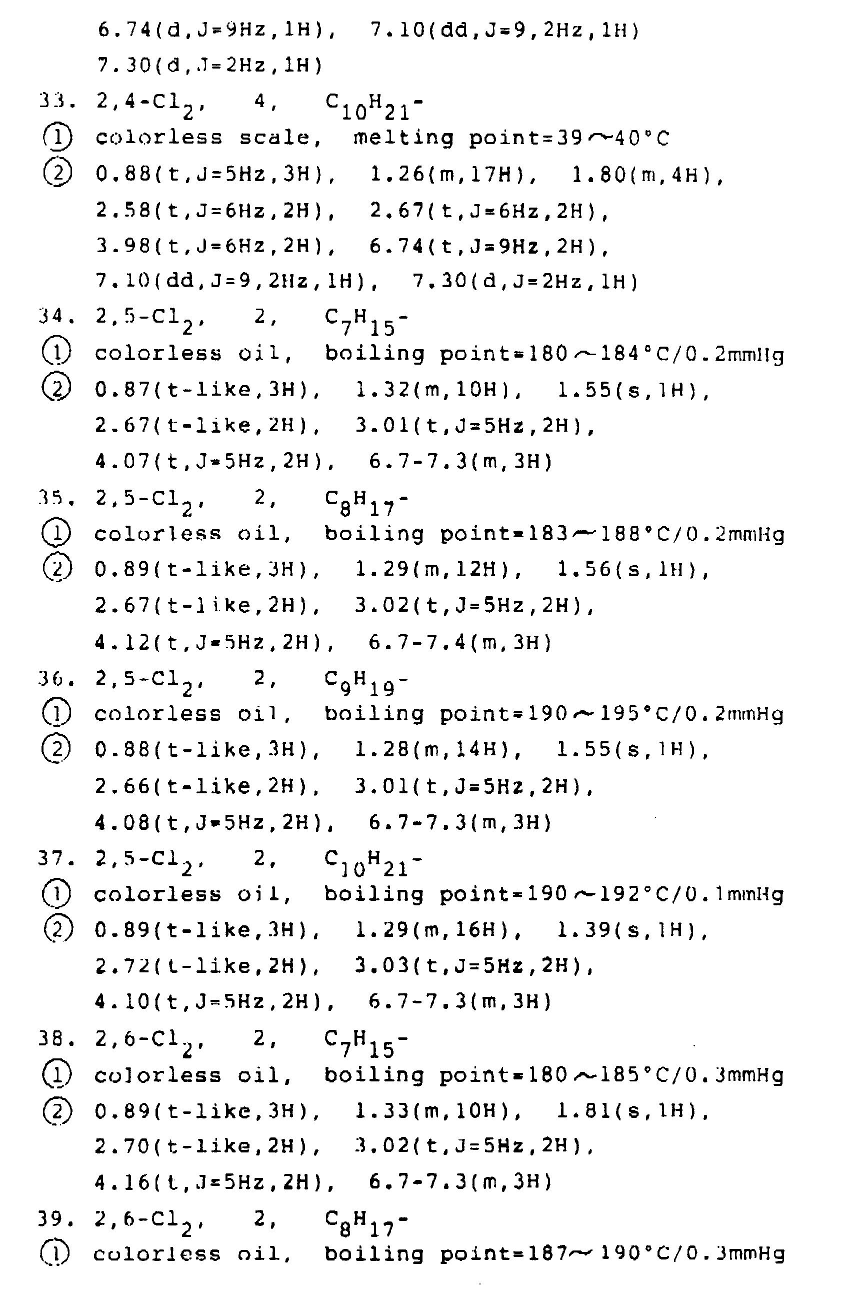

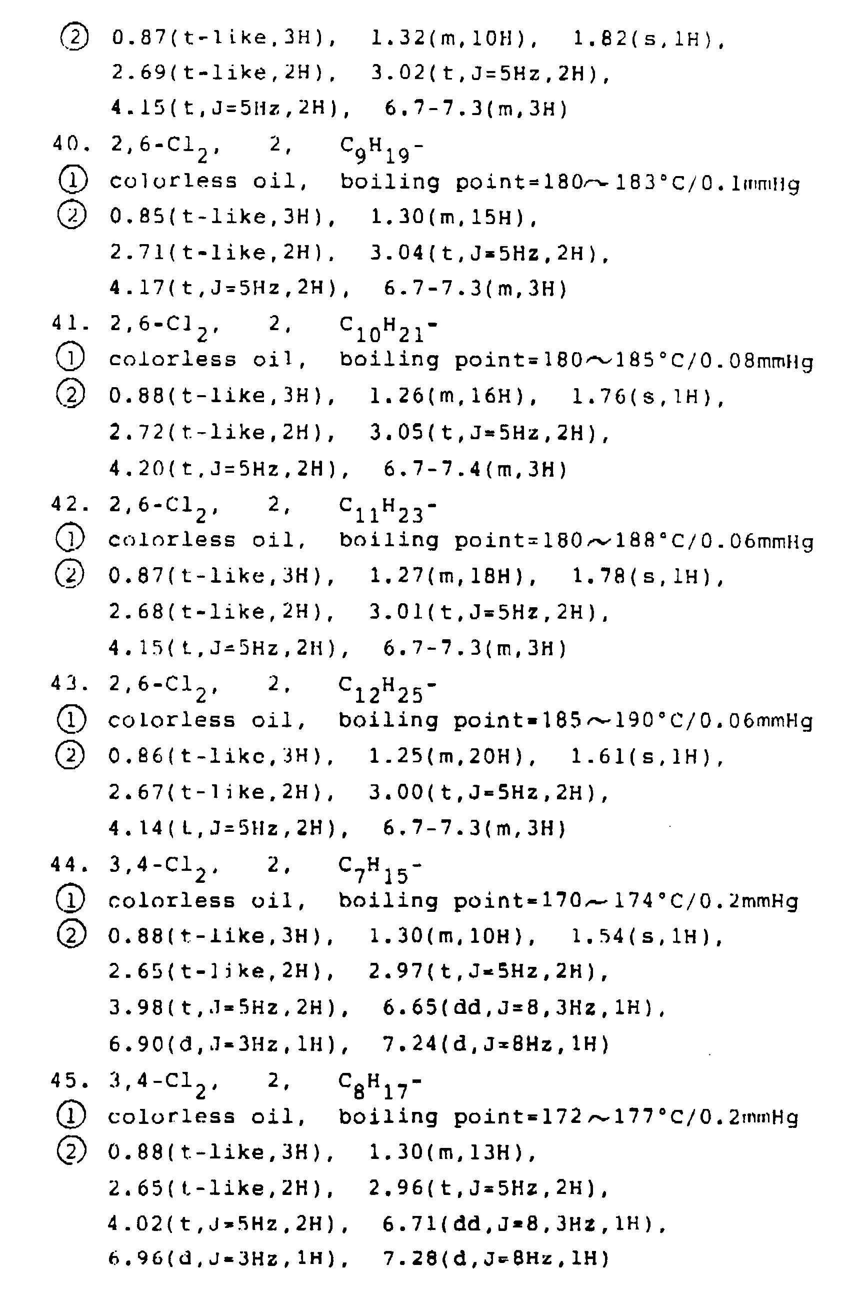

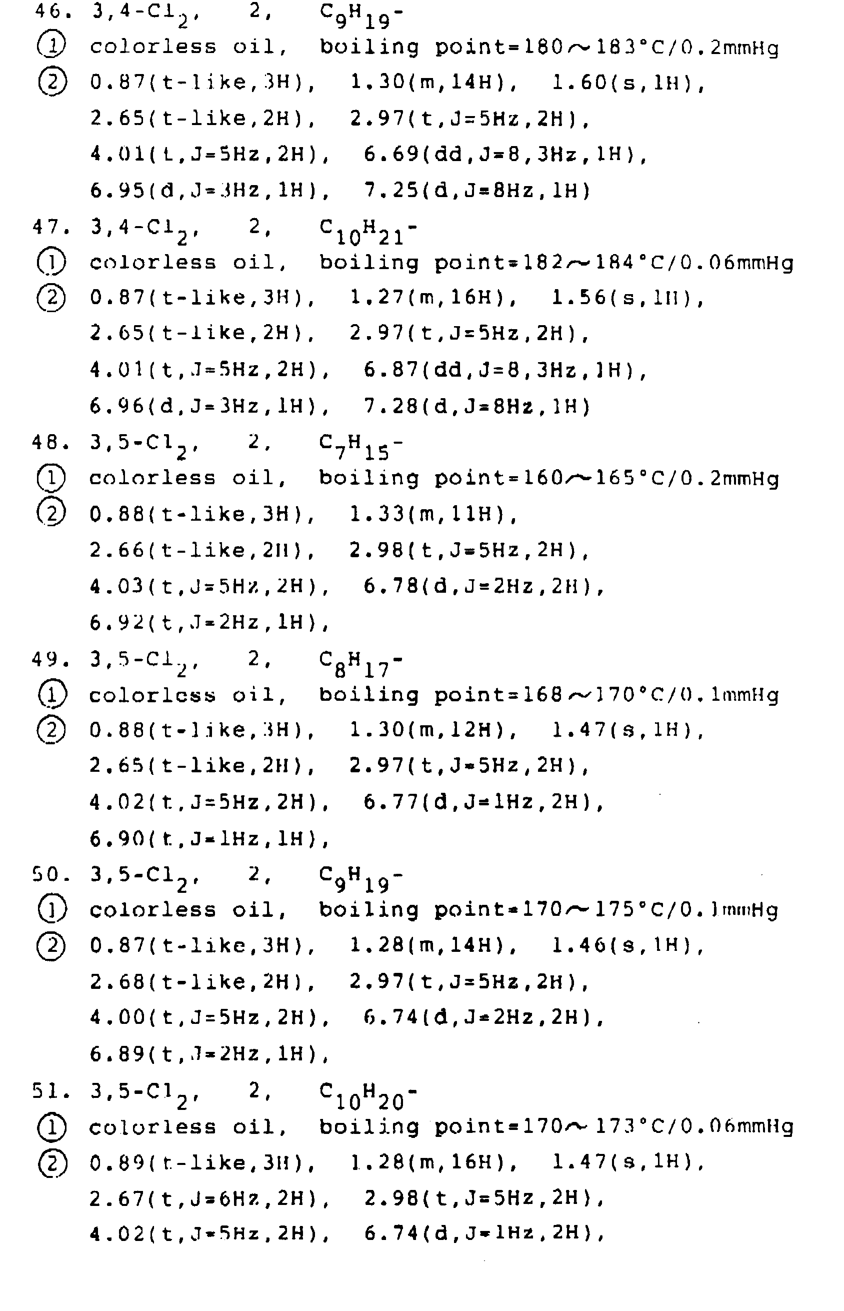

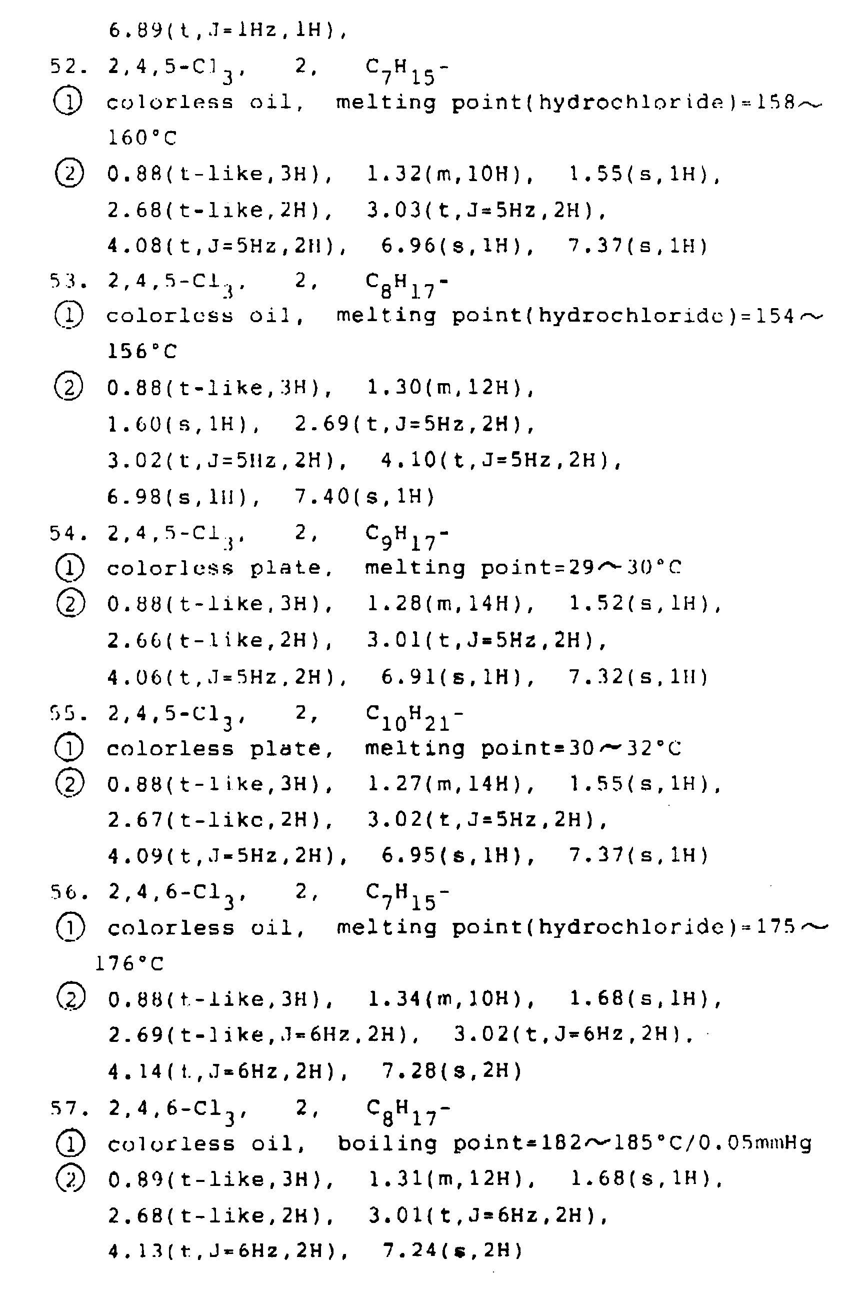

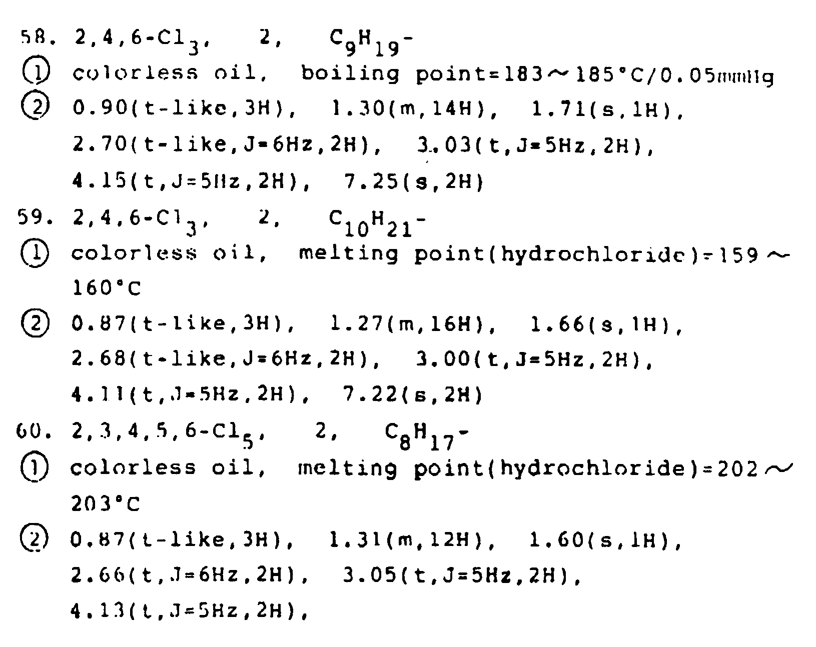

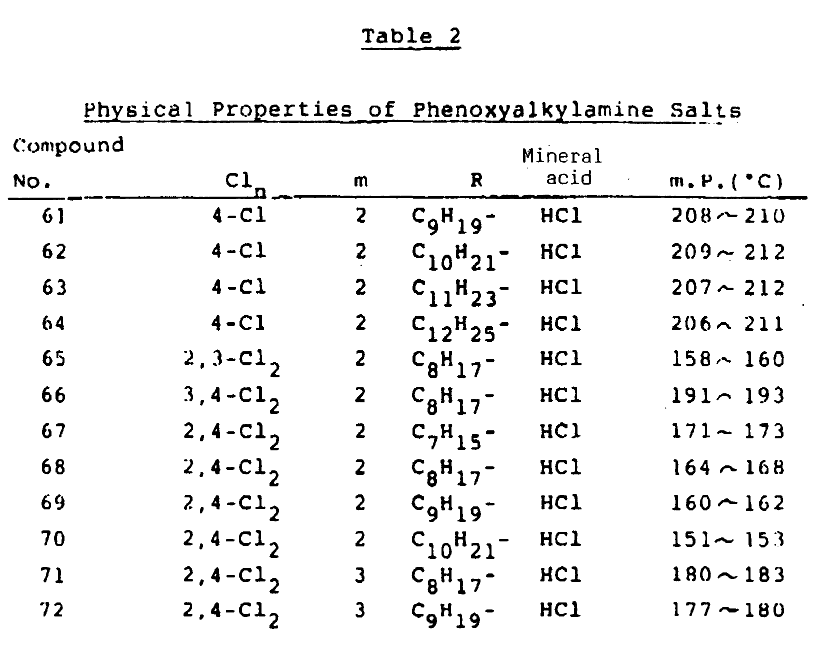

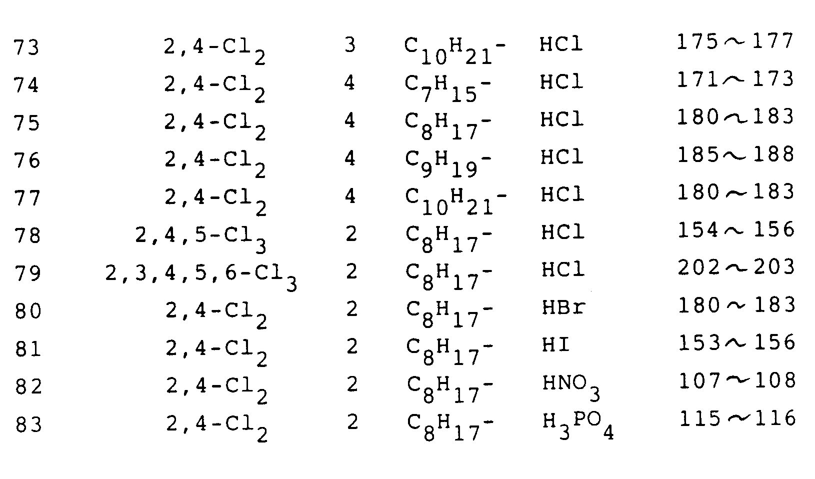

The active ingredients defined herein (phenoxyalkylamine represented by general formula I and the phenoxyalkylamine salt) have strong bacteriostatic and bactericidal actions to bacteria belonging to the genus Xanthomonas such as bacteria causing citrus canker and bacteria belonging to the genus Corynebacterium such as bacteria causing tomato canker, and also to bacteria causing blights in agricultural and horticultural plants.

Each of the active ingredients defined herein is chemically stable and can be preserved for a long time.

Accordingly, because of the strong bacteriostatic and bactericidal actions and the good stability characteristics of the active ingredient, the agricultural and horticultural bactericide of the present invention has a very high practical utility.

Each of the active ingredients of the agricultural and horticultural bactericide of the present invention has a broad antibacterial spectrum, and therefore, the agricultural and horticultural bactericide of the present invention is effective for controlling a variety of blights caused by various pathogenic bacteria, such as citrus canker, bacterial leaf blight of rice, bacterial shot hole of peach, black rot of cabbage, bacterial blight of lettuce, bacterial spot of melon, leaf blight of soy bean, tomato canker and soft rot of Chinese cabbage.

The phenoxyalkylamine salt is advantageous over the phenoxyalkylamine as the active ingredient because recovery, and hence preparation, is easy, the antibacterial action is stronger, the stability is higher and phytotoxicity is lower.

The agricultural and horticultural bactericide of the present invention can be formed into a preparation of an agricultural and horticultural agent, especially a bactericide, such as a wettable powder, a liquid, an emulsifiable liquid, a flowable (sol) preparation, a powder, a driftless (DL) dust or a granule by using the novel compound of the present invention according to customary procedures.

The carrier to be used for such preparations is not particularly critical, and any of carriers customarily used in this field can be used.

As typical instances of the solid carrier, there can be mentioned mineral powders such as kaolin, bentonite, clay, talc and vermiculite, plant powders such as wood meal, starch and crystalline cellulose, and polymeric compounds such as a petroleum resin, polyvinyl chloride, a ketone resin and dammar gum. As typical instances of the liquid carrier, there can be mentioned water, alcohols such as methyl alcohol, ethyl alcohol, n-propyl alcohol, isopropyl alcohol, butanol, ethylene glycol and benzyl alcohol, aromatic hydrocarbons such as toluene, benzene, xylene, ethylbenzene and methyl naphthalene, halogenated hydrocarbons such as chloroform, carbon tetrachloride, dichloromethane, chloroethylene, monochlorobenzene, trichlorofluoromethane and dichlorofluoromethane, ethers such as ethyl ether, ethylene oxide and dioxane, ketones such as acetone, methylethylketone, cyclohexanone and methylisobutylketone, esters such as ethyl acetate, butyl acetate and ethylene glycol acetate, acid amides such as dimethylformamide and dimethylacetamide, sulfoxides such as dimethylsulfoxide, alcohol ethers such as ethylene glycol monomethyl ether and ethylene glycol monoethyl ether, aliphatic and alicyclic hydrocarbons such as n-hexane and cyclohexane, gasolines of the industrial grade such as petroleum ether and solvent naphtha, paraffins, and petroleum fractions such as kerosene and gas oil.

Various surface active agents can be used. As typical instances of the surface active agent, there can be mentioned nonionic surface active agents such as polyoxyethylene alkyl ether and polyoxyethylene alkyl ester, anionic surface active agents such as alkyl benzene-sulfonate and alkyl sulfate, cationic surface active agents such as laurylamine and stearyltrimethyl ammonium chloride, and amphoteric surface active agents such as betaine type carboxylic acid and sulfuric acid esters.

The content of the active ingredient in a preparation as mentioned above is not particularly critical, but from the practical viewpoint, the content of the active ingredient is generally about 0.001 to about 95% by weight (expressed as the compound of general formula I; the same will apply hereinafter) and preferably about 0.01 to about 90% by weight. Practically, in case of a powder, a DL dust and a granule, the content of the compound of the present invention is about 0.01 to about 5% by weight, and in case of a wettable powder, a liquid and an emulsifiable liquid, the content of the compound of the present invention is about 1 to about 75% by weight.

The so-formed preparation, for example, a powder, a driftless dust or a granule, is directly applied, and a wettable powder, a liquid, an emulsifiable liquid or a flowable agent is applied after it has been diluted with water or an appropriate solvent.

The bactericide used according to the present invention can be used in combination with another agricultural and horticultural bactericide, fungicide, a herbicide, an insecticide and a plant growth modifier and also with a fertilizer.

The bactericide of the present invention is dispersed on the leaves or stems of a plant either directly or after dilution, or the bactericide is applied to the water surface, into water, to the soil surface or into interior of the soil.

The application rate of the bactericide of the present invention depends on the kind of the objective disease, the degree of the disease, the kind of the objective plant, the application location, the application method and the kind of the preparation, and it is difficult to specify the application rate simply. However, in case of a powder, driftless dust or granule the concentration of the active ingredient is 3% by weight), the applied amount is 2 to 6 kg per 10 acres. In case of a wettable powder, liquid, emulsifiable liquid or flowable agent (the concentration of the active ingredient is 20% by weight), 0.05 to 3 kg of the active ingredient is used in the state diluted with 100 to 500 R of water per 100 ares.

The compound of the present invention, especially the mineral acid salt, has a strong antibacterial action, a high stability and is not phytotoxic and therefore the application range is broad and the compound of the present invention can be used for not only curing but also preventing blights in plants.

The present invention will now be described in detail with reference to the following examples that by no means limit the scope of the invention.

### Example 1

### Synthesis of N-(2-(2,4-dichlorophenoxy)ethyl)-N-n-octylamine (compound No. 22)

In 20 ml of ethanol was dissolved 2.70 g (10.0 millimoles) of 2-(2,4-dichlorophenoxy)ethyl bromide, and 1.06 g (10.0 millimoles) of anhydrous sodium carbonate and 3.87 g (30.0 millimoles) of n-octylamine were added to the solution. The mixture was heated and refluxed for 6 hours on an oil bath. After cooling, the reaction mixture was poured into 100 ml of water and extracted with 20 ml of chloroform three times.

The organic layer was dried with magnesium sulfate and the solvent was removed by distillation, and the residue was purified by the silica gel column chromatography (developing agent: ethanol/chloroform = 4/9) to obtain 2.67 g (the yield was 84%) of compound No. 22 in the form of a colorless oil.

The physical properties of the obtained compound were as follows.

13C-NMR (CDCl₃):
14.03(q), 22.62(t), 27.26(t), 29.24(t), 29.48(t), 30.09(t), 31.80(t), 48.51 (t), 49.79(t), 69.27(t), 114.43(d), 123.91 (s), 125.86(s), 127.42(d), 129.83(d), 153.18(s)ppm
Mass spectrum:
m/e 317(M⁺, 0.11%), 318(M⁺ + 1, 0.19%), 319(M⁺ + 2, 0.07%), 142(100%), 44(88%).
Infrared absorption spectrum (oil film method):
v max 2920^{s}, 2830^{s}, 1585W, 1475^{s}, 1285^{s}, 1250^{s}, 1100^{m}, 800s, 735° cm-1
Ultraviolet absorption spectrum (ethyl alcohol):
\ max 204(30,300), 231 (8,300), 285(2,000), 293 (1,770)nm

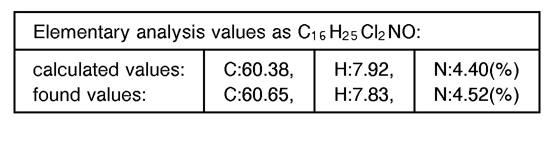

### Example 2

### N-(2-(3,4-dichlorophenoxy)ethyl)-N-n-octylamine (compound no. 45)

In 20 ml of ethanol was dissolved 2.06 g (7.63 millimoles) of 2-(3,4-dichlorophenoxy)ethyl bromide, and 1.06 g (10.0 millimoles) of anhydrous sodium carbonate and 3.48 g (27.0 millimoles) of n-octylamine were added to the solution. The mixture was heated arid refluxed on an oil bath for 6 hours. After cooling, the reaction mixture was poured into 100 ml of water and extracted with 20 ml of chloroform three times.

The organic layer was dried with magnesium sulfate and the solvent was removed by distillation, and the residue was purified by the silica gel column chromatography (developing agent: hexane/ethyl ace- tate/ethanol = 70/26.5/3.5) to obtain 1.97 g (the yield was 82%.) of compound No. 45 in the form of a colorless oil.

The physical properties of the obtained compound were as follows.

13C-NMR (CDCl₃):
14.03(q), 22.62(t), 27.32(t), 29.24(t), 29.48(t), 30.15(t), 31.80(t), 48.57(t), 49.88(t), 68.21 (t), 114.43(d), 116.44(d), 123.97(s), 130.50(d), 132.72(s), 157.85(s)ppm
Mass spectrun:
m/e 317(M⁺, 3.3%), 318(M⁺ + 1, 1.1%), 319(M⁺ + 2, 1.8%) 142(100%), 44(66%).
Infrared absorption spectrum (oil film method):
^{v max} 2920^{vs}, 2850^{s}, 1595^{s}, ^{1565m,} 1455^{vs}, 1280^{s} 1225^{s}, 1120^{s}, 1035^{s}, ^{1020m} cm-₁
Ultraviolet absorption spectrum (ethyl alcohol):
λ max 204(31,700), 232(8,190), 284(1,700), 292(1,570)nm

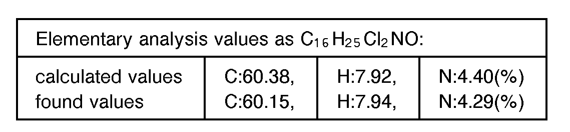

### Example 3

Synthesis of N-(2-pentachlorophenoxyethyl)-N-n-octylamine (compound No. 60)
1) In 45 ml of dimethylformamide was dissolved 8,66 g (30.0 millimoles) of sodium pentachlorophenox- ide, and 50 ml of dibromoethane was added to the solution and the mixture was heated at 100 ° C for 8 hours.

The reaction mixture was poured into 250 ml of a saturated aqueous solution of sodium carbonate and extracted with chloroform, and the organic layer was washed with water and a saturated aqueous solution of sodium chloride and dried with magnesium sulfate. The solvent was removed by distillation, and the obtained solid was sufficiently washed with methanol to obtain 3.58 g (the yield was 32%) of 2-pentachlorophenoxyethyl bromide in the form of a colorless solid.

The physical properties of the obtained compound were as follows:
Melting point: 71-74 ° C
¹H-NMR (CDCl₃):
6 3.64(t, J = 6Hz, 2H), 4.28(t, J = 6Hz, 2H)ppm.
2) In 20 ml of ethanol were dissolved 1.12 g (3.00 millimoles) of 2-pentachlorophenoxyethyl bromide prepared in 1) above and 1.29 g (10.0 millimoles) of n-octylamine, and 0.50 g of anhydrous sodium carbonate was added to the solution and the mixture was heated and refluxed for 6 hours.

After cooling, the reaction mixture was poured into 100 ml of water and extracted with chloroform. The organic layer was dried with magnesium sulfate and the solvent was removed by distillation, and the residue was purified by the silica gel column chromatography (developing agent: hexane/ethyl ace- tate/ethanol = 70/29/1) to obtain 0.91 g (the yield was 71 %) of compound No. 60 in the form of a colorless oil.

The physical properties of the obtained compound were as follows.

Infrared absorption spectrum (oil film method):
v max 3220^{W}, 2900^{s}, 2850^{vs}, 1340^{s}, 1125^{m}, 1025^{m} 1015^{vs}, 750^{m}, 705^{m} cm⁻¹
Ultraviolet absorption spectrum (ethyl alcohol):
λ max 213(87,000), 226^{sh}(20,500), 238^{sh}(10,300), 291^{sh}(540), 296(670)nm

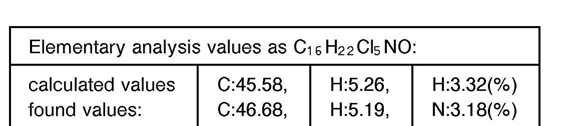

### Example 4

### Synthesis of compound No. 68

In 40 ml of ether was gradually dissolved 3.18 g (10.0 millimoles) of N-2-(2,4-dichlorophenoxy)ethyl-N-n-octylamine (compound No. 22), and under ice cooling, a slight excess of hydrochloric acid gas was blown into the solution to form a crystal.

The formed crystal was recovered by filtration and sufficiently washed with dry ether to obtain 3.47 g (the yield was 98%) of compound No. 68 in the form of a white solid (having a melting point of 164 to 168 ° C).

### Example 5

### Synthesis compound No. 83

In 40 ml of ethanol was dissolved 3.18 g (10 millimoles) of N-2-(2,4-dichlorophenoxy)ethyl-N-n-octylamine (compound No. 22), and under ice cooling, 1.27 g (11.0 millimoles) of 85% phosphoric acid was added to the solution to form a crystal. The formed crystal was recovered by filtration, sufficiently washed with ether and dried to obtain 4.02 g (the yield was 96%) of compound No. 83 in the form of a white solid (having a melting point of 115 to 116 ° C).

### Preparation Example 1 (Wettable Powder)

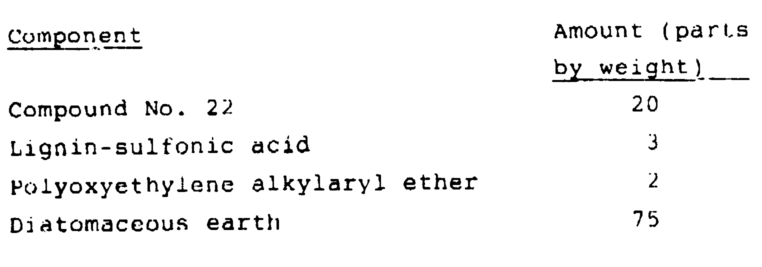

The foregoing components were homogeneously mixed to obtain a wettable powder comprising 20% by weight of the active ingredient.

### Preparation Example 2 (Powder)

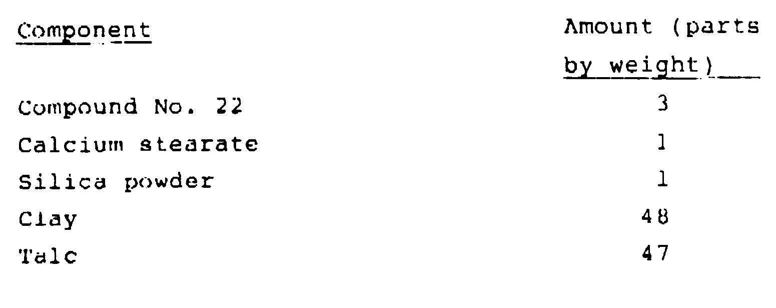

The foregoing components were homogeneously mixed to obtain a powder comprising 3% by weight of the active ingredient.

### Preparation Example 3 (Wettable Powder)

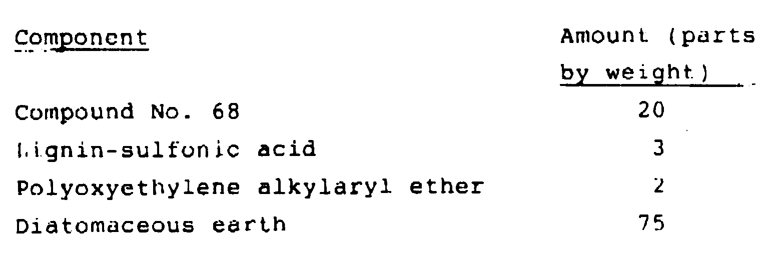

The foregoing components were homogeneously mixed to obtain a wettable powder comprising 20% by weight of the active ingredient.

### Preparation Example 4 (Powder)

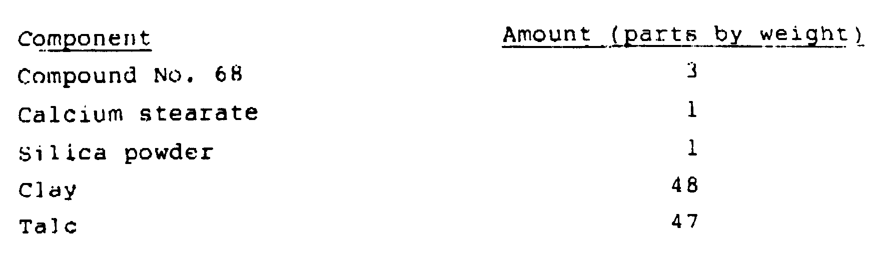

The foregoing components were homogeneously mixed to obtain a powder comprising 3% by weight of the active ingredient.

### Test 1 (Antibacterial Test to Pathogenic Bacteria to Plants)

Antibacterial actions of the phenoxyalkylamine and phenoxyalkylamine salt to various pathogenic bacteria to plants were examined.

More specifically, the bacterium causing black spot of cabbage, Xanthomonas campestris pV. campestris, the bacterium causing citrus canker, X. campestris pV. citri, the bacterium causing bacterial leaf blight of rice, X. campestris pV. oryzae, and the bacterium causing tomato canker, Corynwbacterium michiganense pV. michiganense, were used as the bacterium to be tested, and the action of inhibiting the growth of the bacterium on an agar plate was examined.

The sample compound was added to a peptone-added potato extract medium, and a 2-times dilution system having a maximum concentration of 100 ppm was prepared and the culture medium was cast into a Petri dish to form an agar plate.

The agar plate was inoculated with the bacterium to be tested and incubation was carried out at 28 _{°} C for 2 days, and the growth of the bacterium was checked.

The obtained results are shown in Table 3.

The compounds of the present invention showed a strong antibacterial action to all of the pathogenic bacteria.

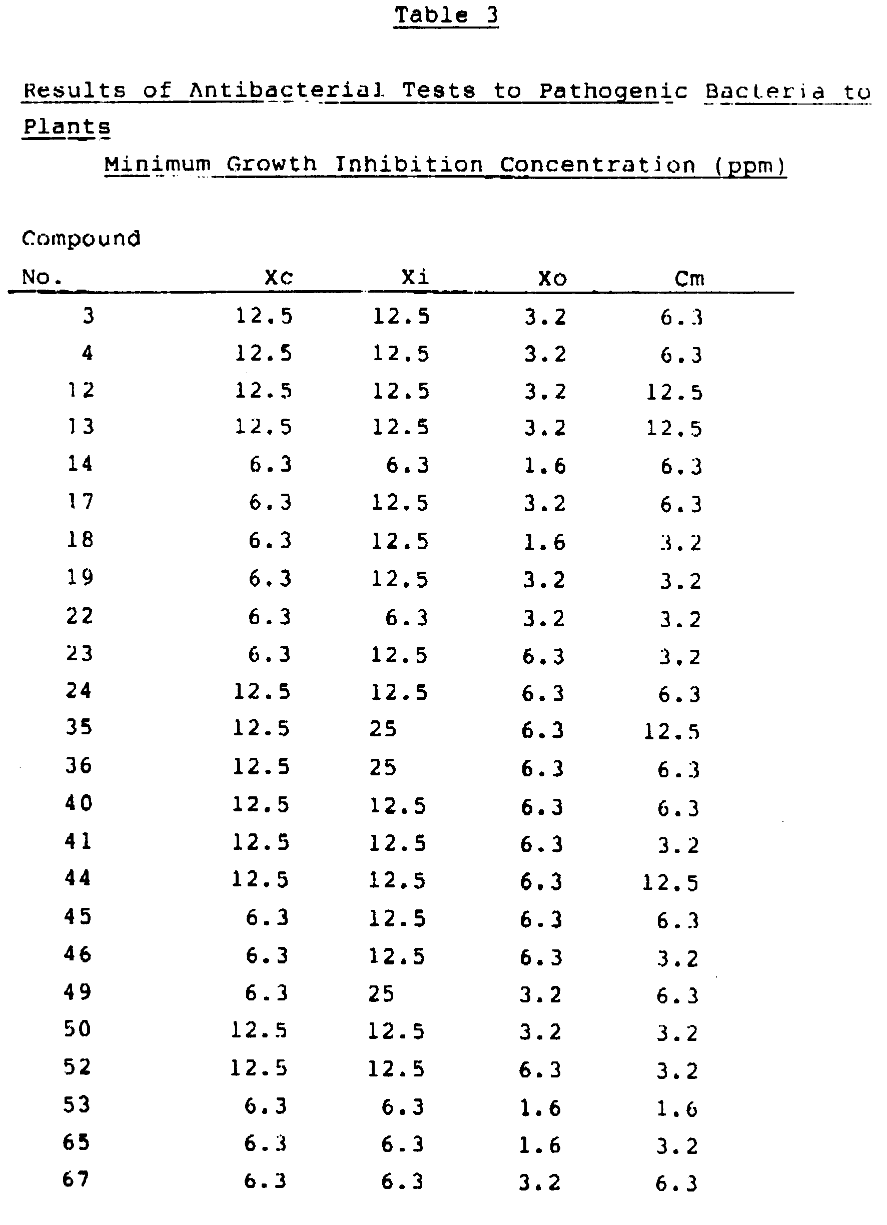

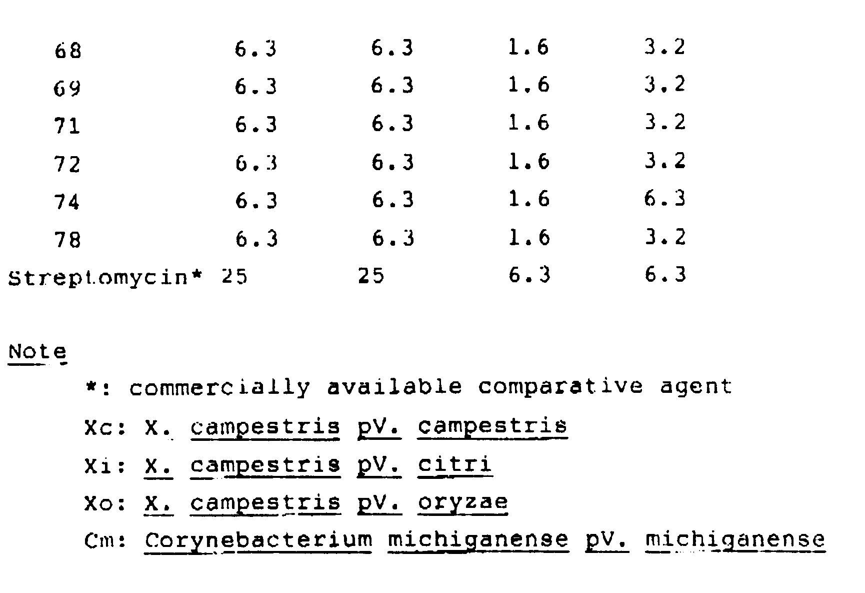

### Table 2 (Test of Preventing Citrus Canker)

Leaf pieces having a square shape having a side of about 1 cm were cut out from summer orange leaves and immersed in a chemical solution having a predetermined concentration for 20 minutes. The leaf pieces were taken out from the chemical solution and were then air-dried. Then, the leaf pieces were inoculated with a suspension of cells of the bacterium causing citrus Canker (about 10⁸ cells per mt) by using a needle.

The inoculated leaf pieces were placed in a Petri dish on which a wet filter paper was spread, and incubation was carried out at 28 ° C for 10 days and the outbreak of the disease was checked. The disease attack ratio was calculated according to the following formula: 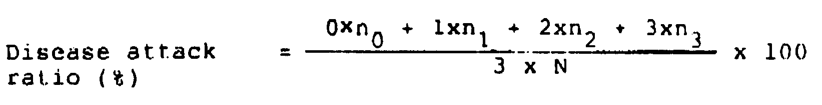
wherein no represents the number of leaf pieces having a disease severity index of 0 (no disease), ni represents the number of leaf pieces having a disease severity index of 1 (slight disease), n₂ represents the number of leaf pieces having a disease severity index 2 (medium disease), n₃ represents the number of leaf pieces having a disease severity index of 3 (violent disease), and N represents the total number of the examined leaf pieces.

Furthermore, the state of occurrence of the phytotoxicity was visually examined.

### The obtained results are shown in Tables 4-(1) and 4-(2).

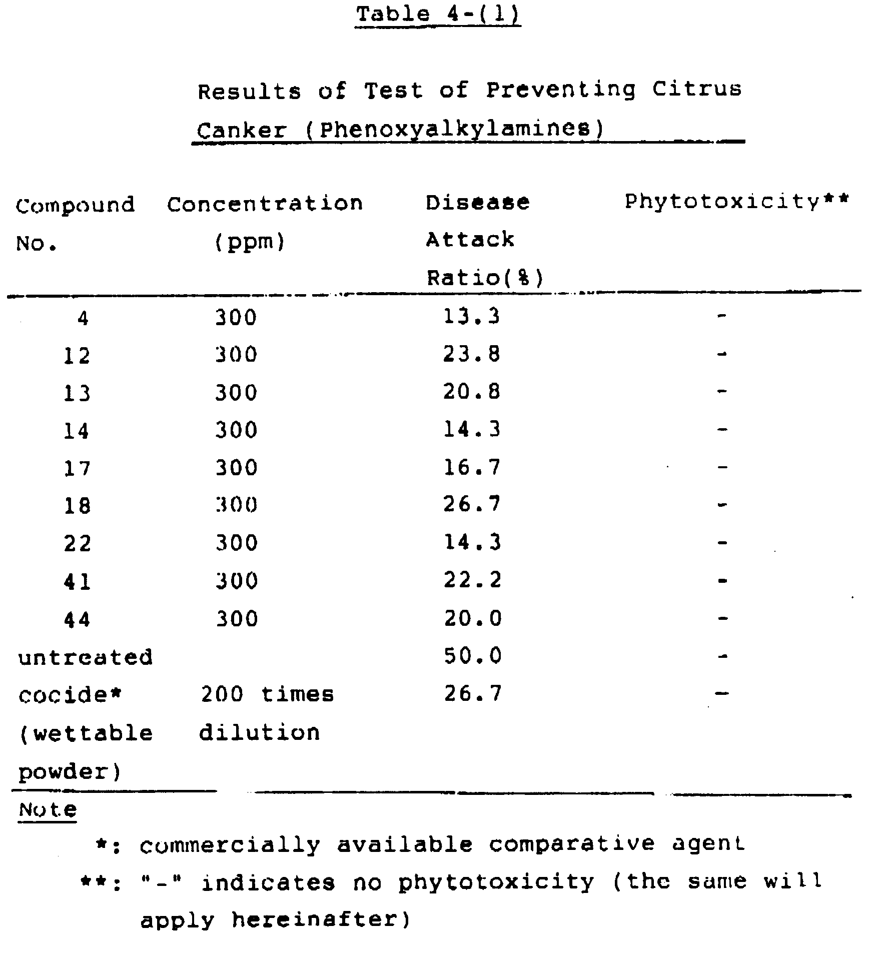

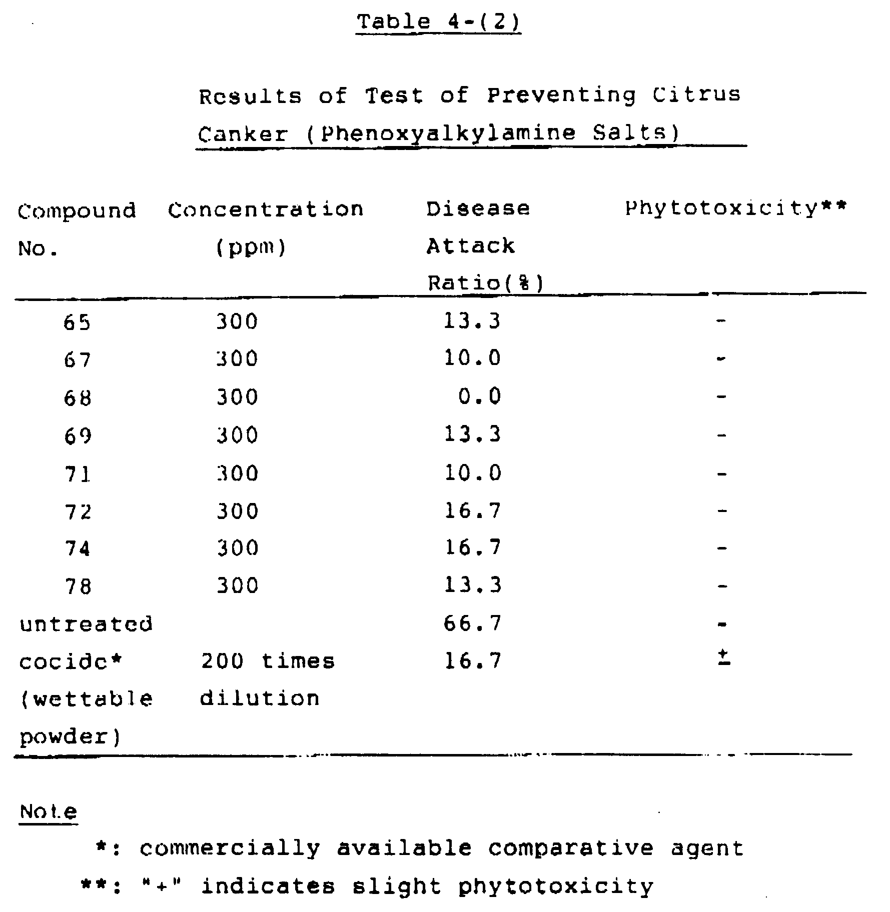

### Test 3 (Test of Controlling Bacterial Leaf Blight of Rice)

An aqueous solution containing the sample compound at a predetermined concentration was scattered to rice plants of the 5-leaf stage (variety: Hoshihikari) grown in a pot having a diameter of 6 cm.

After one day, the rice plants were shear-inoculated with a cell suspension of the bacterium causing bacterial leaf blight of rice, which had a concentration of 10⁸ cells per mt.

When 3 weeks passed from the inoculation, the lengths of disease spots were measured, and the control values was calculated according to the following formula: 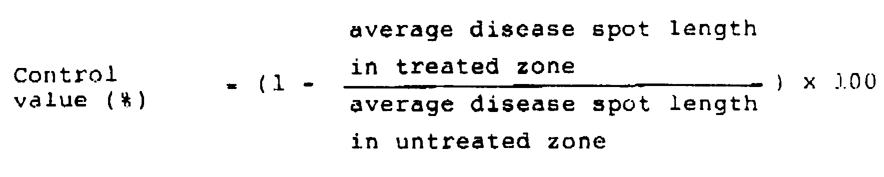 The obtained results are shown in Table 5.

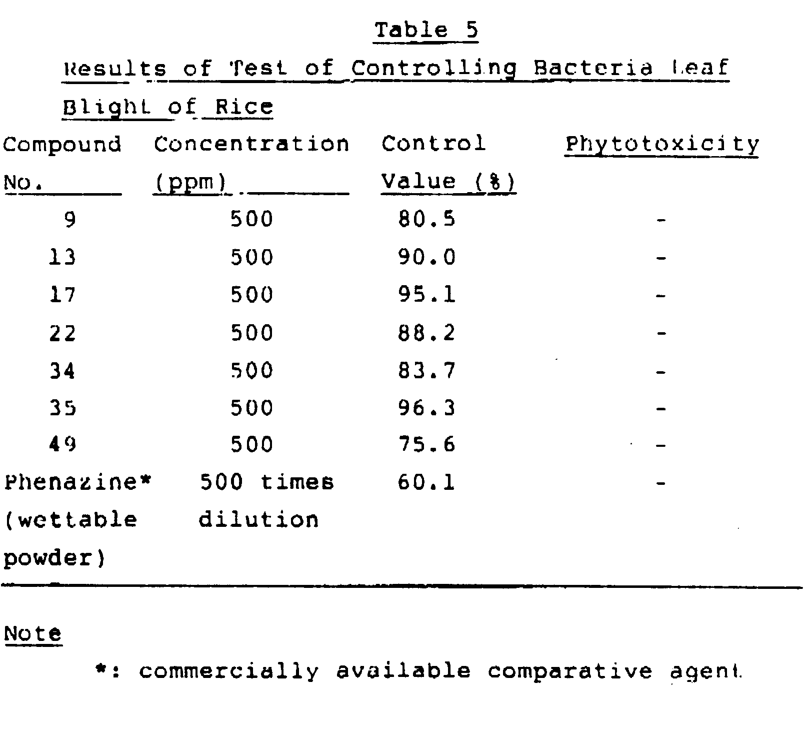

### Test 4 (Test of Controlling Dry Rot)

Radish disks having a diameter of 2 cm and a thickness of 1 cm were prepared and immersed in an aqueous solution containing the sample compound at a predetermined concentration for 1 hour.

The radish disks were taken out from the aqueous solution and air-dried. A bacterium suspension was dropped on central portions of the disks and the disks were maintained at 28 ° C for 24 hours. The disease attack degree was examined and the control values were calculated according to the following formula: 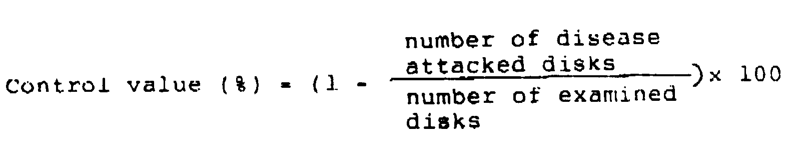 The obtained results are shown in Tables 6-(1) and 6-(2).

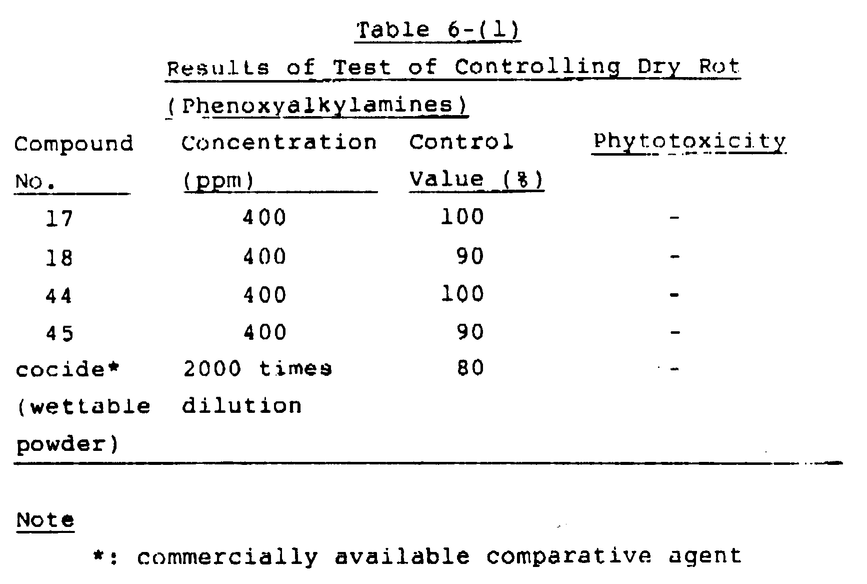

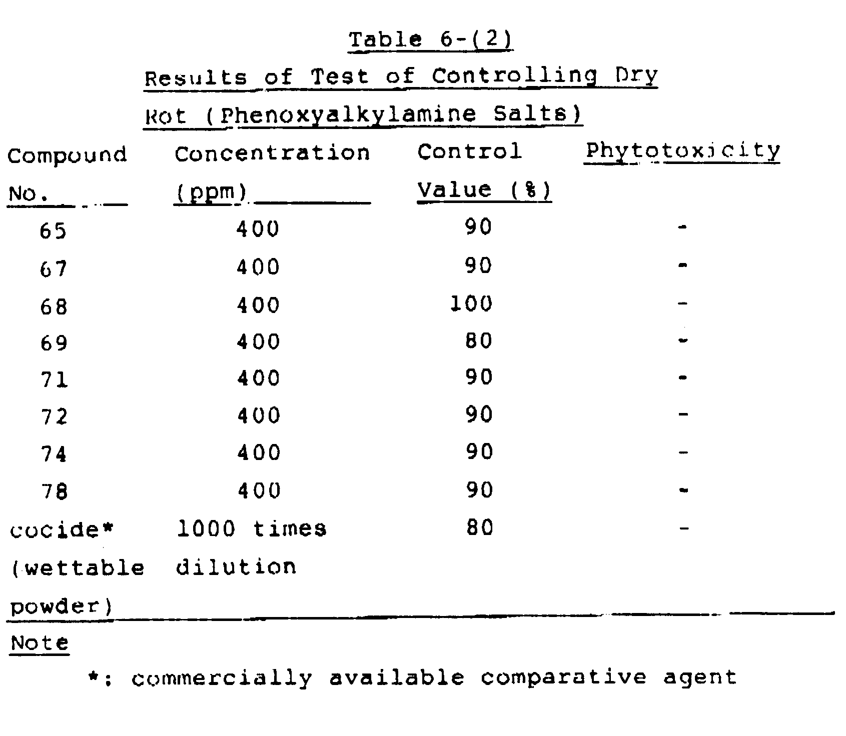

### Test 5 (Test of Stability to Ultraviolet Rays)

A watch glass was charged with 0.5 ml of a methanol solution containing 2% by weight of compound No. 68, and the watch glass was air-dried.

The watch glass was placed 20 cm below a sterilizing lamp (10 W) and irradiated with rays from the lamp for a predetermined time.

Then, 0.5 ml of methanol was added into the watch glass to form a solution, and the minimum growth inhibition concentration was measured.

The obtained results are shown in Table 7.

From the results shown in Table 7, it is seen that in case of compound No. 22, the minimum inhibition concentration drastically increased after 3 days' irradiation, and compound No. 22 was stable during 2 days' irridiation. In contrast, the minimum inhibition concentration was not changed even after 7 days' irradiation by the sterilizing lamp in case of compound No. 68, and it was confirmed that compound No. 68 was more stable to ultraviolet rays.

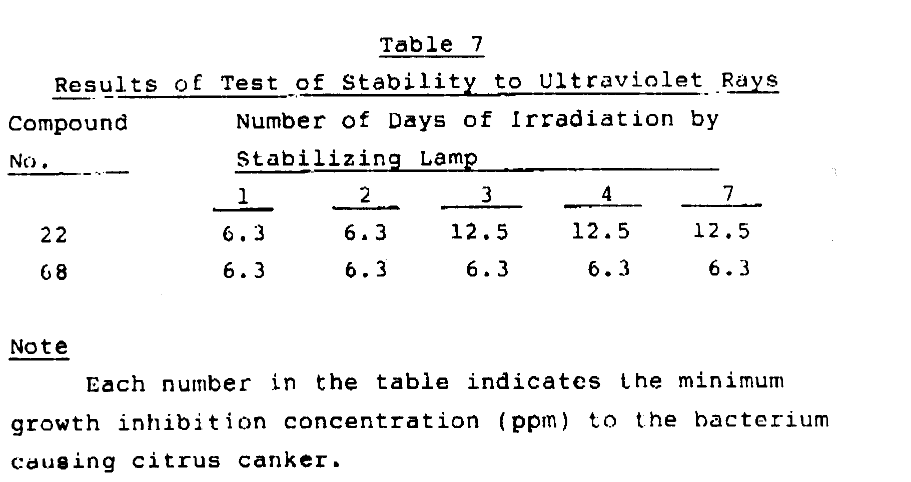

### Test 6 (Phytotoxicity Test)

Compound No. 67 was dissolved at a predetermined concentration, and the solution was scattered to plants of the 3-leaf stage. After 1 week, the phytotoxicity was examined.

Rice, wheat, tomato and cucumber were used as the test plant.

The obtained results are shown in Table 8.

In case of compound No. 22, the phytotoxicity was observed at a high concentration of 1,000 ppm.

In contrast, compound No. 68 did not show any phytotoxicity to any of the plants even at a high concentration of 1,000 ppm.

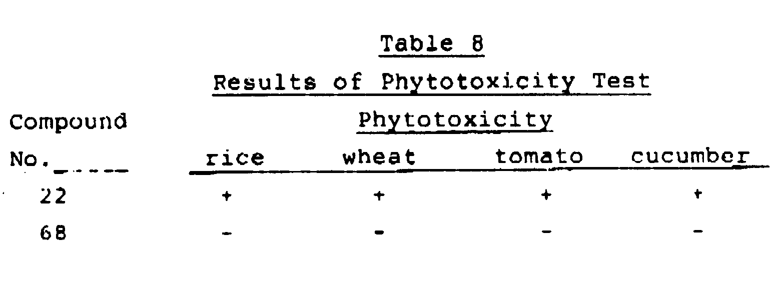

## Claims

1. Use of agricultural or horticultural bactericide comprising an active ingredient which is a phenoxyalkylamine of formula (I) 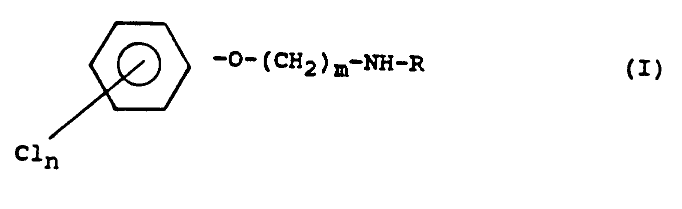 wherein R is a linear saturated hydrocarbon having 7 to 12 carbon atoms, n is an integer of from 1 to 5 and m is an integer of from 2 to 4, or a mineral acid salt thereof, for preventing or curing a plant disease.

2. Use according to claim 1 wherein the phenoxyethylamine is of formula (III)

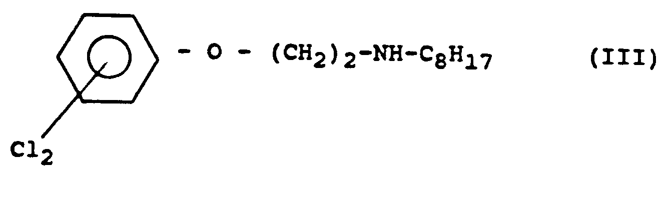

3. Use according to claim 1 or 2 wherein the active ingredient is a hydrogen chloride salt of a compound of formula (I).

4. Use according to claim 1, 2 or 3 wherein the plant disease is citrus canker, bacterial leaf blight of rice, bacterial shot hole of peach, black rot of cabbage, bacterial blight of lettuce, bacterial spot of melon, leaf blight of soy bean, tomato canker or soft rot of Chinese cabbage.

5. Use of a bactericide as defined in claim 1, 2 or 3 for killing plant pathogenic bacteria selected from Xanthomonas campestris, Corynebacterium michiganense and Erwinia carotovora.

## Patentansprüche

1. Verwendung eines Bakterizids für die Landwirtschaft oder den Gartenbau, umfassend einen Wirkstoff, der ein Phenoxyalkylamin ist der Formel (I) 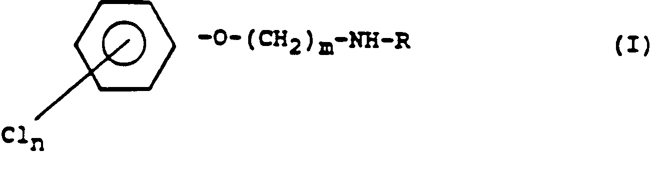 wobei R ein linear gesättigter Kohlenwasserstoff mit 7 bis 12 Kohlenstoffatomen ist, n eine ganze Zahl von 1 bis 5 ist und m eine ganze Zahl von 2 bis 4 ist, oder ein Mineralsäuresalz davon, zur Verhütung oder Heilung einer Pflanzenkrankheit.

2. Verwendung nach Anspruch 1, wobei das Phenoxyethylamin der Formel (111) 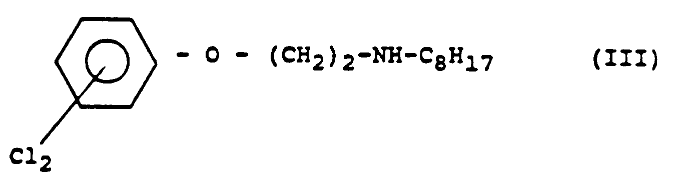 entspricht.

3. Verwendung nach Anspruch 1 oder 2, wobei der Wirkstoff ein Hydrochloridsalz einer Verbindung der Formel (I) ist.

4. Verwendung nach Anspruch 1, 2 oder 3, wobei die Pflanzenkrankheit Citruskrebs, bakterielle Fleckkrankheit bei Pfirsichen, Schwarztrockenfäule bei Kohl, echter Mehltau am Salat, bakterielle Fleckkrankheit bei Melonen, Blattbrand von Sojabohnen, Tomatenkrebs oder Weichfäule von Chinakohl ist.

5. Verwendung eines Bakterizids nach Anspruch 1, 2 oder 3 zum Töten von Pflanzen-pathogenen Bakterien, ausgewählt aus Xanthomonas campestris, Corynebacterium michiganense und Erwinia carotovora.

## Revendications

1. Utilisation en agriculture ou en horticulture d'un bactéricide comprenant un principe actif qui est une phénoxyalkylamine de formule (I) 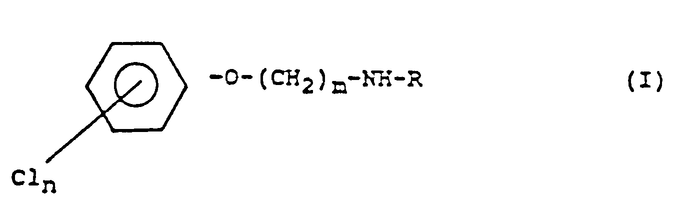 où R est un radical hydrocarboné saturé linéaire ayant 7 à 12 atomes de carbone, n est entier de 1 à 5 et m est un entier de 2 à 4, ou l'un de ses sels d'addition d'acide minéraux, pour prévenir ou traiter les maladies des plantes.

2. Utilisation selon la revendication 1, où la phénoxy éthylamine est de formule (III).

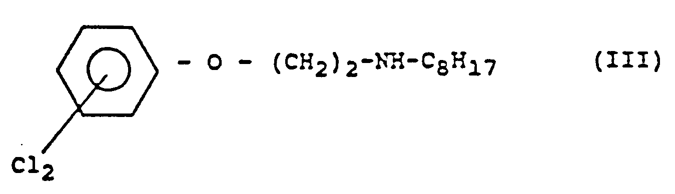

3. Utilisation selon la revendication 1 ou 2 où le principe actif est un sel d'acide chlorhydrique du composé de formule (1).

4. Utilisation selon la revendication 1, 2 ou 3 où la maladie de la plante est le chancre des agrumes, la flétrissure généralisée bactérielle des feuilles de riz, la perforation bactérielle des feuilles du pêcher, la pourriture du chou, la flétrissure bactérielle de la laitue, bactériose du melon, la flétrissure des feuilles de soja, le chancre des tomates, ou la pourriture molle du chou chinois.

5. Utilisation du bactéride tel que défini dans les revendications 1, 2 ou 3 pour tuer les bactéries pathogènes chez les plantes choisies parmi Xanthomonas campestris, Corynebacterium michiganense et Erwinia Carotovora.
